# EUROPÄISCHE PATENTSCHRIFT

(11) **EP 1 656 213 B1**
(45) Veröffentlichungstag und Bekanntmachung des Hinweises auf die Patenterteilung: **21.03.2012**
(21) Anmeldenummer: 04738152.0
(22) Anmeldetag: 18.08.2004
(51) Int. Cl.: B01F 13/00, B05C 17/005, A61B 17/00, B65D 81/32, A61F 2/46

(54) **VORRICHTUNG UND VERFAHREN ZUM AUFBEWAHREN, MISCHEN UND AUSTRAGEN VON KOMPONENTEN**
DEVICE AND METHOD FOR THE STORAGE, MIXING AND DISPENSING OF COMPONENTS
DISPOSITIF ET PROCEDE DE CONSERVATION, DE MELANGE ET D'APPLICATION DE CONSTITUANTS

(30) Priorität: 21.08.2003 CH 142303
(43) Veröffentlichungstag der Anmeldung: 17.05.2006
(73) Patentinhaber: Medmix Systems AG, 6343 Rotkreuz (CH)
(72) Erfinder: KELLER, Wilhelm, A., CH-6402 Merlischachen (CH)
(74) Vertreter: Detken, Andreas
(86) Internationale Anmeldenummer: PCT/CH2004/000517
(87) Internationale Veröffentlichungsnummer: WO 2005/018830

(56) Entgegenhaltungen:
- EP-A- 0 292 472
- WO-A-00/35506
- WO-A-01/85070
- WO-A-95/20408
- DE-C- 632 579
- DE-U1- 20 214 747
- US-A- 3 048 192
- US-B1- 6 402 364

## Beschreibung

Die vorliegende Erfindung bezieht sich auf eine Vorrichtung und Verfahren zum Aufbewahren, Mischen und Austragen von Komponenten, mit Mitteln zum Vermischen einer ersten Komponente mit einer zweiten, flüssigen Komponente und Austragen der Mischung, gemäss Oberbegriff von Patentanspruch 1. Bei der ersten Komponente kann es sich um pulverförmiges, granulatförmiges oder poröses Knochenersatzmaterial oder Knochenzement handeln oder ähnliche, in der Medizin oder Zahnmedizin verwendbare Materialien.

Eine Vorrichtung gemäss Oberbegriff ist aus der US-A-6 402 364 bekannt, die einen Dispenser für gefärbte kosmetische Stoffe offenbart, wobei eine zentral angeordnete Spritze über einen Drehverschluss mit einem Kanal entweder von einem oder vom anderen daneben angeordneten Behälter Farben ansaugen kann, um diese zu mischen und auszutragen. Eine solche Anordnung ist für den Medizinalbereich nicht geeignet.

Weitere Vorrichtungen sind auf dem Markt erhältlich und enthalten in der Regel einen Behälter, in welchem das pulverförmige Material aufbewahrt ist und in welches die flüssige Komponente beigegeben wird, woraufhin die Komponenten vermischt werden und das Gemisch durch einen Kolben ausgetragen wird.

Durch die örtlich getrennten Lagerung von mindestens zwei Komponenten kann die Lagerhaltung unübersichtlich und die Beimischung der kleineren, flüssigen Komponente problematisch werden, z.B. durch Verwechslung.

Es sind weitere Vorrichtungen bekannt, bei denen die Komponenten getrennt gelagert werden, beispielsweise wird die kleinere, flüssige Komponente hinter dem Kolben oder Stössel der Kammer der grösseren, pulverförmigen Komponente gelagert. Bei solchen Anordnungen ist im Allgemeinen ein Ventil zwischen den Komponenten angeordnet, durch welches die flüssige Komponente in die pulverförmige Komponente austreten kann. Diese Lösung hat den Nachteil, dass kein kontrollierbarer Transfer der Komponenten möglich ist. Stellvertretend für eine Anzahl von Dokumenten zu diesem Stand der Technik sei die US-A-3 370 754 genannt.

Aus der EP-0 292 472 ist ein Set zur Bereitstellung und Applikation eines Gewebeklebstoffes bekannt, wobei jeweils vier Spritzenkörper paarweise über ein Kupplungsstück zu einer Einheit vereinigt sind. Das Kupplungsstück weist je einen Konus zur Aufnahme eines entsprechenden konischen Teils an.der Spritze auf. Es wird jeweils nur eine Verbindung zwischen zwei nebeneinanderliegende Spritzen offenbart, jedoch ohne Ventilanordnung, so dass die Anwendungsmöglichkeiten beschränkt sind.

Aus der WO 00 35506 ist ein Behälter für ein Pulver und ein Behälter für eine Flüssigkeit bekannt, um Knochenzement bereitzustellen, wobei zwingend ein Anschluss für Vakuum vorgesehen sein muss und wobei die Verbindung zwischen den beiden Behältern im Prinzip immer offen ist und der Behälter mit der Flüssigkeit kurz vor dem Mischen geöffnet wird. Es kann jedoch keine wahlweise Verbindung zwischen den Behältern hergestellt werden.

Es ist von diesem bekannten Stand der Technik ausgehend Aufgabe der vorliegenden Erfindung, eine Vorrichtung und ein Verfahren zum Lagern, Mischen und Austragen von Komponenten, insbesondere von pulverförmigen, granulatförmigen, porösen und flüssigen Materialien vorzusehen, die bei vollständiger Trennung der Komponenten bei der Lagerung eine einfache Handhabung und Anordnung der Behälter ermöglicht und eine einfache Steuerung des Einbringens der zweiten, flüssigen Komponente in die erste Komponente erlaubt sowie eine grosse Vielfalt an Anwendungsmöglichkeiten wie externes ansaugen oder Einbringen einer Flüssigkeit in die Austragvorrichtung bietet. Die Vorrichtung, die diese Aufgabe löst, ist in den Patentansprüchen 1 und 15 definiert.

Die Erfindung wird im Folgenden anhand von Zeichnungen von Ausführungsbeispielen näher erläutert.
- Fig. 1: zeigt, teilweise geschnitten und in perspektivischer Sicht, ein erstes Ausführungsbeispiel einer erfindungsgemässen Vorrichtung,
- Fig. 2: zeigt schematisch ein Detail der Lösung gemäss Fig. 1,
- Fig. 3: zeigt im Schnitt von Fig. 2 ein erstes Ausführungsbeispiel eines Ventils,
- Figuren 4-7: zeigen verschiedene Verfahrensschritte beim Überführen einer Komponente in die andere, sowie beim Mischen und Austragen der aufbereiteten Mischung mit der Vorrichtung nach Fig. 1,
- Fig. 8: zeigt eine Ausführungsvariante mit Kolben ohne Stössel für die zweite, flüssige Komponente,
- Fig. 9: zeigt ein zweites Ausführungsbeispiel des Austragkolbens, ohne Stössel,
- Fig. 10: zeigt ein weiteres Ausführungsbeispiel mit einem Vakuumanschluss,
- Fig. 11: zeigt ein weiteres Ausführungsbeispiel einer Vorrichtung gemäss Erfindung,
- Fig. 12: zeigt die Vorrichtung von Fig. 11 in einer anderen Auslasskonfiguration und Stellung eines Stössels,
- Fig. 13: zeigt in perspektivischer Sicht ein weiteres Ausführungsbeispiel einer Vorrichtung zum Ansaugen einer Flüssigkeit, Mischen mit einem Pulver und Austragen des Gemisches,
- Fig. 14: zeigt die Vorrichtung von Fig. 13 teilweise im Längsschnitt kurz vor dem Gebrauch,
- Fig. 15: zeigt die Vorrichtung von Fig. 14 nach dem Transfer der Flüssigkeit,
- Fig. 16: zeigt in einer Ausschnittvergrösserung den Ein/ Auslassbereich als Dreiweg-Ventilanordnung der Spritze von Figur 13, und einen Schnitt A gemäss der Linie A - A,
- Fig. 17: zeigt in einer anderen Schnittebene den Ein/ Auslass von Fig. 16, und einen Schnitt B gemäss der Linie B - B,
- Fig. 18: zeigt in einer weiteren Schnittebene den Ein/ Auslass von Fig. 16, und einen Schnitt C gemäss der Linie C - C,
- Fig. 19: zeigt in einem vierten Schnitt D die geschlossene Stellung des Ventils, ,
- Fig. 20: zeigt ein weiteres Auslasszubehörteil,
- Fig. 21: zeigt eine Zweikomponenten-Spritze mit getrennten Vorratsbehältern, und
- Fig. 22: zeigt die zusammengefügte Zweikomponenten-Spritze von Fig. 21.
- Fig. 23: zeigt eine Dreikomponenten-Spritze mit getrennten Vorratsbehältern, und
- Fig. 24: zeigt die zusammengefügte Dreikomponenten-Spritze von Fig. 23.

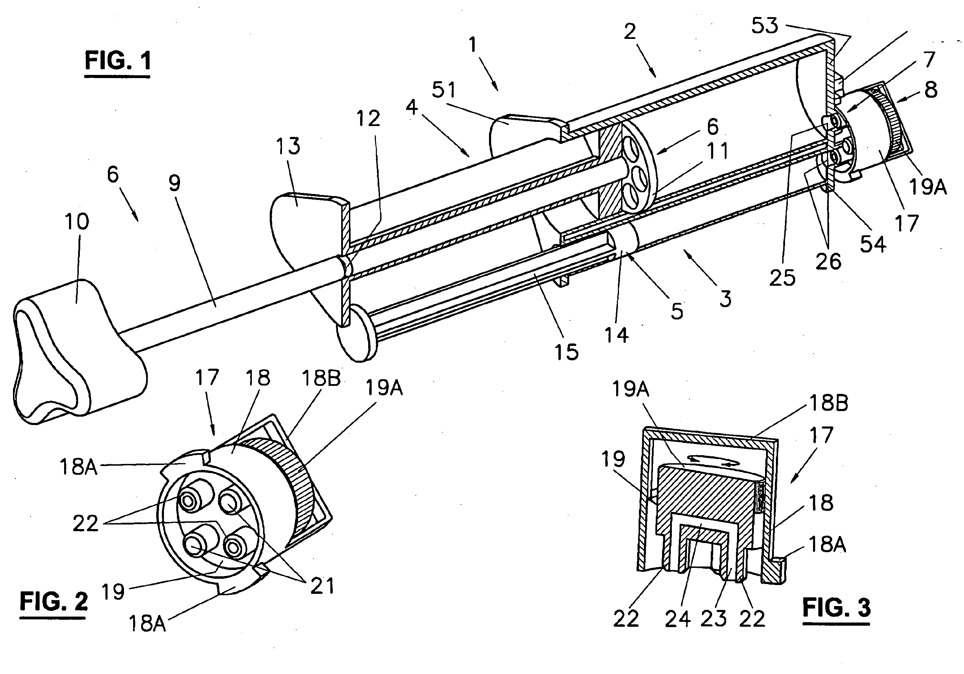

Fig. 1 zeigt schematisch den grundsätzlichen Aufbau einer erfindungsgemässen Vorrichtung 1 in einer ersten Ausführungsform, mit einem Behälter 2 für pulverförmiges Material, einem Behälter 3 für ein flüssiges Material, eine Kolbeneinheit 4 zum Austragen des gemischten Materials, eine Kolbeneinheit 5 zum Überführen der flüssigen Komponente in den Behälter 2 und eine Mischeinrichtung 6 zum Durchmischen der pulverförmigen und flüssigen Komponente im Behälter 2 sowie einen Transferbereich 7 zwischen dem Auslassbereich 26 des zweiten Behälters 3 und dem Einlass 25 des ersten Behälters 2. Die Behälter weisen im hinteren Bereich einen Rückhalteflansch 51 auf.

Statt eines pulverförmigen Materials kann als erste Komponente auch ein flüssiges, granulatförmiges, poröses oder pastöses Material verwendet werden, dem vor dem Austragen eine Flüssigkeit zugemischt wird. Im Folgenden wird jedoch beispielhaft auf ein pulverförmiges Material Bezug genommen und der Einfachheit halber der erste Behälter als Pulverbehälter und der zweite oder dritte Behälter als Flüssigkeitbehälter bezeichnet. Auch können die Austragvorrichtungen handelsübliche Kartuschen sein. Ausserdem wird hier unter dem Begriff "Vermischen" auch das Auflösen einer Komponente in einer anderen verstanden.

Der Behälter 2 für die erste Komponente, ein pulverförmiges Material 27 und der Behälter 3 für die zweite, flüssige Komponente 28 sind, durch einen Luftspalt getrennt, nebeneinander angeordnet, so dass die Diffusionsgefahr von einem Behälter in den anderen praktisch beseitigt ist. Das nebeneinander Anordnen bedingt eine Verbindung zwischen der flüssigen Komponente und dem pulverförmigen Material, wobei im Transferbereich 7 Mittel zum wahlweise Verschliessen oder Öffnen der Verbindung zwischen den Behältern vorhanden sein muss. Vorteilhafterweise wird dies durch eine weiter unten beschriebene Ventilanordnung 8 realisiert.

Die Mischeinrichtung 6 besteht im Wesentlichen aus einem im Behälter hin- und herbewegbaren Mischstab 9 mit Drehknopf 10 und einem Mischorgan, in diesem Beispiel eine Mischscheibe 11, die entweder gelocht wie in vorliegendem Beispiel und/oder mit am Umfang angeordneten Ausnehmungen, oder sonstwie geeignet gestaltet sein kann. Die Mischscheibe 11 kann mittels des Mischstabes und Drehknopfes sowohl gedreht als auch hin- und herbewegt werden, um eine gute Durchmischung des pulverförmigen Materials mit der flüssigen Komponente zu gewährleisten.

Die Mischeinrichtung wird nach dem,Mischen nicht mehr verwendet und daher weist der Mischstab 9, wie in Fig. 1 eingezeichnet, etwa auf der Höhe der Druckplatte 13 der Kolbeneinheit eine Sollbruchstelle 12 auf. Dort kann das überstehende Mischstabende mitsamt dem Drehknopf abgebrochen werden.

Eine Mischeinrichtung mit durch den Kolben geführten Mischstab, Drehknopf und Mischscheibe kann auch für andere Spritzen oder Austragvorrichtungen verwendet werden, die keine Ventilanordnung aufweisen.

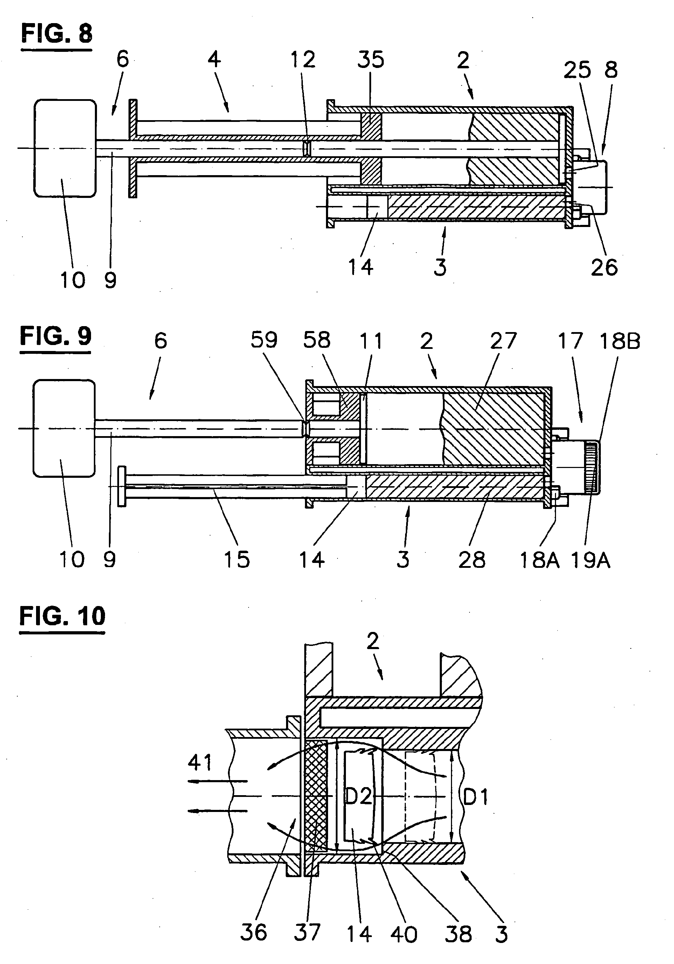

Bezüglich der Kolbeneinheit für die zweite, flüssige Komponente sind im Allgemeinen zwei Varianten denkbar, eine Kolbeneinheit mit Kolben 14 und Stössel 15 oder eine Kolbeneinheit gemäss Fig. 8 mit Kolben 14 alleine, der durch Unterdruck verschiebbar ist.

In den Figuren 1 - 3 ist eine erste Ausführungsform der Ventilanordnung 8 dargestellt, in der sie als Ventilkappe 17 mit einem Gehäuse 18 mit einem Bügel 18B und zwei Bajonettlaschen 18A, die in entsprechende Halterungen 16 und 20 am Gehäuseboden 53 von Behälter 2 und Gehäuseboden 54 von Behälter 3 greifen, ausgebildet ist. Im Innern des Kappengehäuses 18 ist eine Zapfenanordnung 19 mit zwei Paaren Zapfen 21 und 22 angeordnet, die gegenüber dem Gehäuse drehbar ist und einen Knopf 19A aufweist, der durch das Gehäuse ragt. Die Zapfen 21 sind voll und dienen als Verschluss des Transferbereichs 7, während die Zapfen 22 je eine Bohrung 23 aufweisen, die über einen Verbindungskanal 24 miteinander verbunden sind, wie dies aus Fig. 2A hervorgeht.

Je nachdem, welches Zapfenpaar in den Einlass 25 an Behälter 2 und Auslass 26 an Behälter 3 eingeführt wird, besteht entweder eine Verbindung zwischen beiden Behältern, oder nicht. Durch Abnehmen der Kappe und Drehen der Zapfen um 90° sowie Wiederanschliessen mittels der Bajonettverbindung wird wahlweise die Verbindung unterbrochen oder hergestellt.

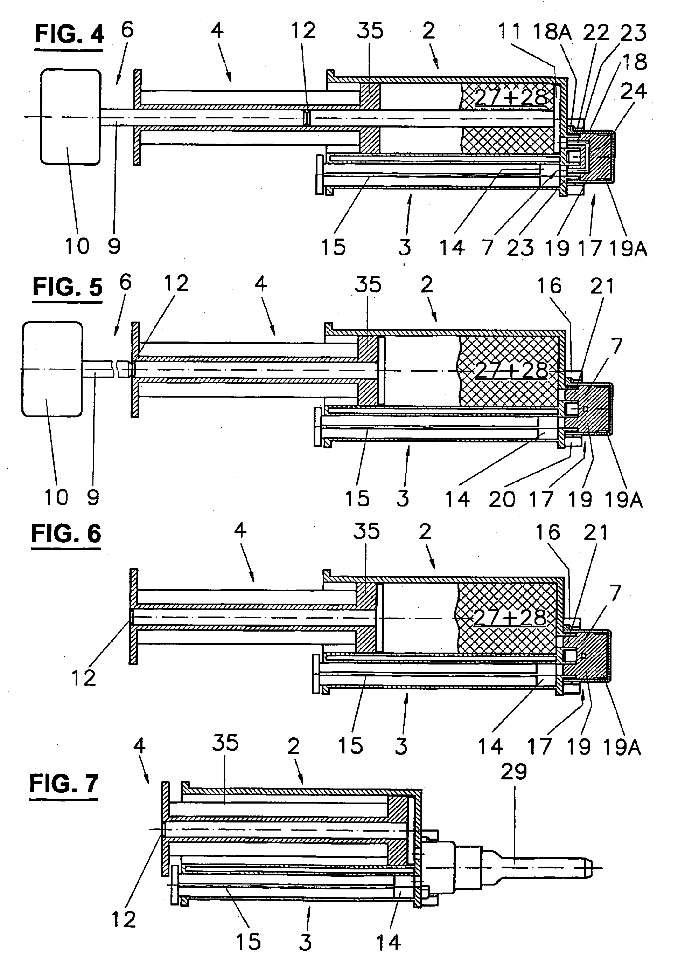

In den Figuren 4 - 7 sind vier verschiedene Phasen der Verwendung einer Vorrichtung nach Fig. 1 dargestellt. Bei der Darstellung von Fig. 4 wurde die Kolbeneinheit 5 betätigt und die Flüssigkeit 28 in den Behälter 2 gedrückt und, nach Umstecken des Ventils von Durchlass auf Verschluss, gemäss Figur 5 mit Hilfe der Mischeinrichtung 6 mit dem Pulver 27 vermischt.

In der Darstellung von Fig. 6 ist das Mischstabende der Mischeinrichtung an der Sollbruchstelle abgebrochen und dann entfernt worden, so dass nach Entfernen der Ventilkappe die Vorrichtung bereit zum Austragen ist. In der Darstellung von Fig. 7 wurde eine Austragspitze 29 angebracht und das Gemisch durch die Kolbeneinheit 4 ausgetragen.

In der Ausführungsvariante gemäss Figur 8 wird der Kolben 14 im Behälter 3 nicht durch einen Stössel sondern durch Unterdruck betätigt. Durch das Herausziehen der in einer mittleren Stellung sich befindlichen Kolbeneinheit 4 mit Kolben 35 wird ein Unterdruck erzeugt, wodurch der Kolben 14 in Behälter 3 Richtung Auslass 26 bewegt wird. Dazu muss eine offene Verbindung zwischen dem Auslass 26 und dem Einlass 25 vorhanden sein. Bei dieser Variante ist es zweckmässig, dass die Wand von Behälter 2 eine Innennut oder Innenwulst und der Kolben 35 entsprechende Mittel aufweist, um zu verhindern, dass er ganz herausgezogen wird.

In einer weiteren Ausführungsvariante gemäss Figur 9 wird der Kolben 58 der Kartusche oder Behälter 2 nicht von einem daran befestigten Stössel beaufschlagt, sondern vom Stössel eines Austraggerätes, und die Sollbruchstelle 59 des Mischstabes 9 ist näher am Kolben. Der Kolben 58 sollte eine genügende axiale Länge haben, damit beim Abbrechen des Mischstabes bei der Sollbruchstelle 59 der Kolben 58 nicht verkippt.

Bei einer Anwendung der Anordnung unter Vakuum gemäss Figur 10 wird eine Vakuumquelle an den leeren Flüssigkeitsbehälter 3 angeschlossen, der in Verbindung mit dem Pulverbehälter 2 steht und diesen unter Vakuum setzt, um unter Vakuum zu mischen. In Fig. 10 ist das Einlassende 36 von Behälter 3 dargestellt, in welches am Abschluss ein Filter 37 eingebaut ist. Der Innendurchmesser D2 des Einlassendes ist grösser als derjenige D1 des übrigen Behälters, so dass ein Absatz 38 entsteht. In Figur 10 ist das Absaugen dargestellt, nachdem Kolben 14, hier mit Dichtlippen 40, beaufschlagt wurde, um die Flüssigkeit in den Behälter 2 zu überführen. Durch das Anlegen des Vakuums, symbolisiert durch die Pfeile 41, wird der Kolben 14 zurückgezogen, bis die Dichtlippen in den erweiterten Bereich D2 gelangen, wodurch die in den Behältern sich befindliche Luft entweichen kann, um bei gewissen Pulvern wie PMMA eine möglichst luftfreie, gute Durchmischung erst zu ermöglichen. Das Filter 37 kann sich auch im Anschlussstutzen für das Vakuum befinden.

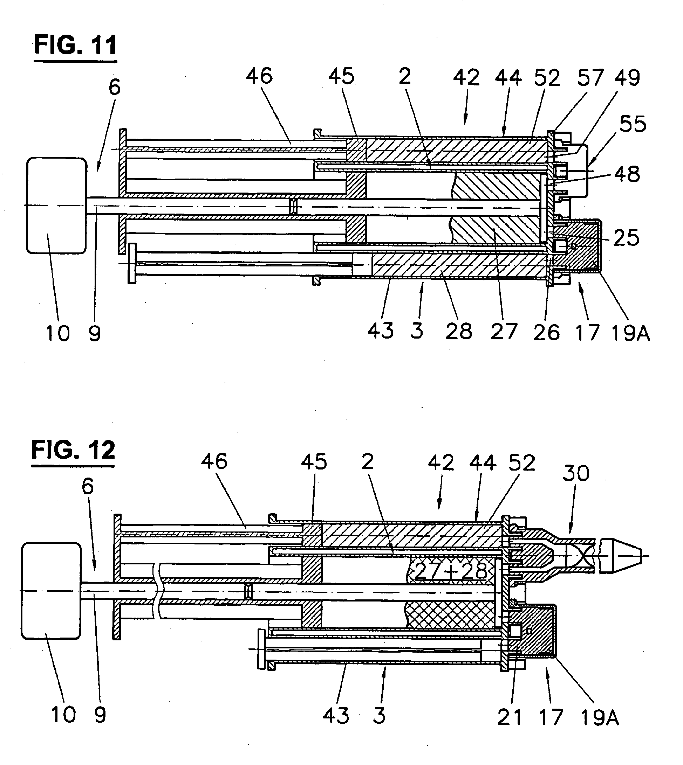

In den Figuren 11 und 12 ist eine Vorrichtung 42 dargestellt, in welcher im Gehäuse 43 neben den beiden Behältern 2 und 3 ein dritter Behälter 44 angeordnet ist. Dieser dritte Behälter 44 mit Kolben 45 und Stössel 46 kann gleich dem Behälter 3 mit Kolben und Stössel ausgebildet sein, jedoch auch eine andere Dimension aufweisen und ist mit einer anderen flüssigen Komponente 52 gefüllt, z.B. ein Hormon, Antibiotika und dergl.

In der Ausgangsstellung von Fig. 11 ist der Auslass 48 für das Gemisch von Behälter 2 und 3 und der Auslass 49 von Behälter 44 mit einem gemeinsamen Verschluss 55 versehen, der die Verbindung unterbricht, solange die in das Pulver überführte erste Flüssigkeit vermischt wird. Zum Austragen der zweiten Flüssigkeit und vom Gemisch wird der Verschluss entfernt und ein Mischer 30 oder ein Zubehörteil am gemeinsamen Auslass 47 angebracht. Dabei können die üblichen Bajonett-Anschlussteile in Gehäuseboden 57 verwendet werden.

In Figur 12 ist angedeutet, dass zuerst die Flüssigkeit aus Behälter 3 in Behälter 2 gebracht wurde und anschliessend das Gemisch aus Behälter 2 und die Komponente aus Behälter 44, um durch den gemeinsamen Mischer ausgetragen zu werden.

Von den Figuren 11 und 12 ausgehend ist es möglich, nicht nur einen zusätzlichen Behälter sondern auch weitere Behälter, analog Behälter 44, um Behälter 2 anzuordnen, die je einen Verschluss der Auslässe und einen gemeinsamen Anschluss vom Auslass des Behälters und dem Auslass von Behälter 2 besitzen.

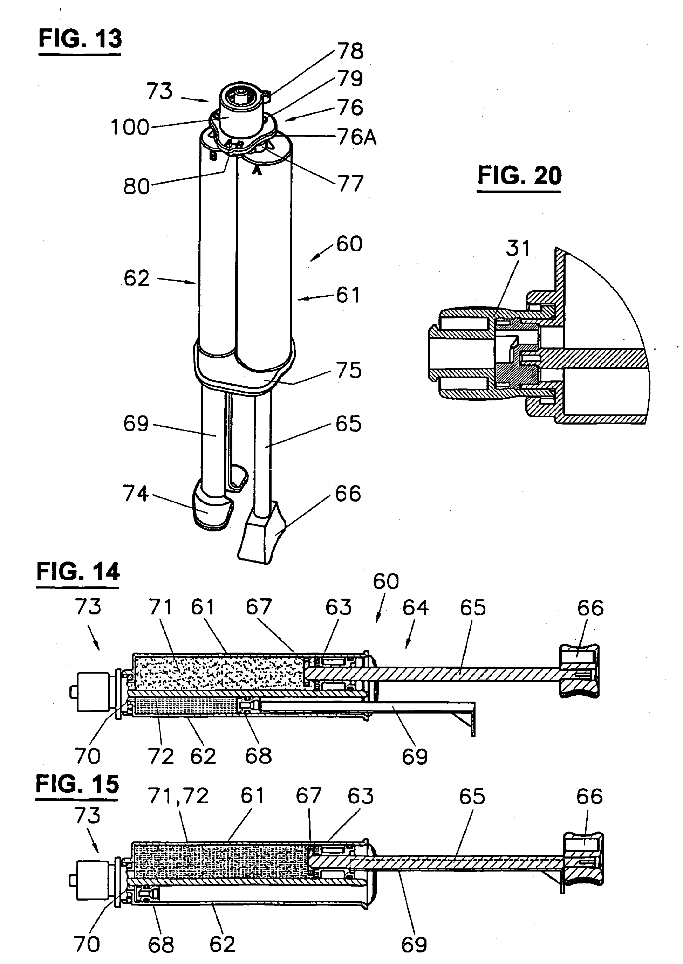

In den Figuren 13 bis 18 ist eine Vorrichtung angegeben, mit der es möglich ist, eine Flüssigkeit, z.B. Blut, anzusaugen, mit einem Pulver zu vermischen und das Gemisch auszutragen. Figur 13 zeigt die Vorrichtung 60 in perspektivischer Sicht, mit dem Pulverbehälter 61, mit "A" beschriftet, für Pulver 71 und einen Flüssigkeitsbehälter 62, mit,"B" beschriftet für eine Flüssigkeit 72. Eingangsseitig ist der Mischstab 65 mit dem Drehknopf 66 und der lose Stössel 69 mit dem Stossflansch 74 sichtbar sowie der Rückhalteflansch 75.

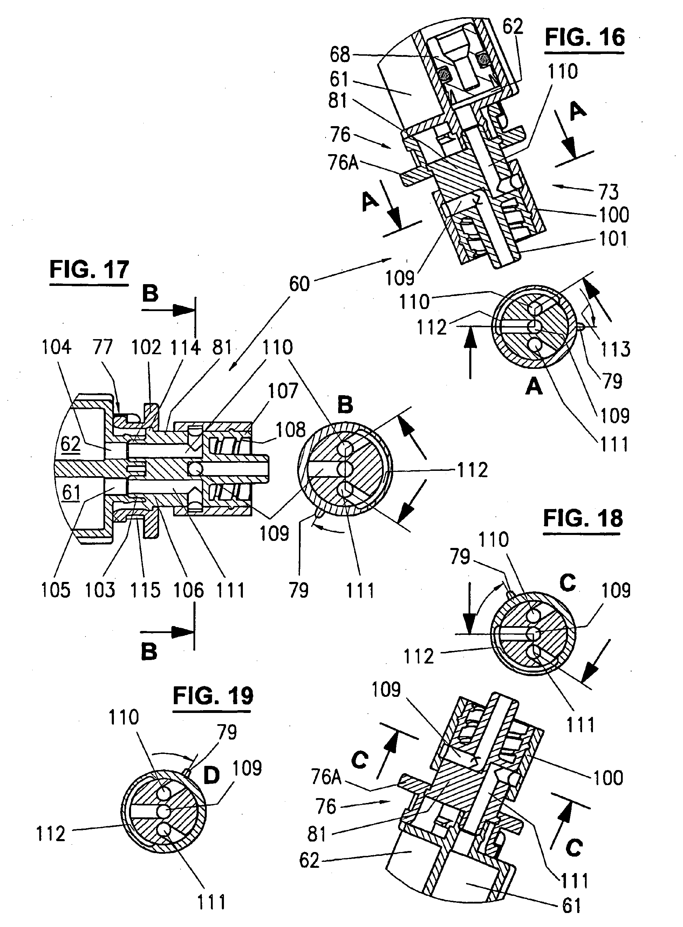

Ausgangsseitig befindet sich die Ventilanordnung 73 mit dem Befestigungsteil 76 mit den Bajonett-Anschlussteilen 77 und dem Drehring 100, auf dem am freien Ende eine Handhabe 78 und bei der Anzeigescheibe 76A des Befestigungsteils 76 ein Zeiger 79 angebracht sind, wobei der Zeiger 79 die jeweilige Stellung des Dreiweg-Ventils auf der Anzeigescheibe 76A anzeigt, zum Beispiel A -- B, Verbindung zwischen den beiden Vorratsbehältern oder A -- O oder B -- O, Verbindung zwischen Behälter A oder Behälter B mit dem Ein/Auslass, oder gemäss Figur 19 die geschlossene Stellung. Zwecks Orientierung beim Aufsetzen der Ventilanordnung weist die Befestigungsteil eine Nase 80 auf.

Die Befestigung der Ventilanordnung kann auch auf andere Weise als mit Bajonettsanschlussteilen erfolgen, z.B. mittels einer drehfesten Schnappverbindung, die auch direkt am Ventilkörper angeordnet sein kann.

Zum Einlass hin ist im Pulverbehälter 61 ein Kolben 63 angeordnet, durch den die Mischeinrichtung 64 mit Mischstab 65, Drehknopf 66 und Mischscheibe 67 geführt ist. Der Flüssigkeitsbehälter 62 ist bei seinem Einlass mit einen Kolben 68 versehen, der durch einen separaten, losen Stössel 69 in Richtung Auslass 70 bewegbar ist.

Bei der Verwendung der Doppelspritze 60 wie in den vorhergehenden Beispielen, d.h.Transfer der Flüssigkeit, Mischen und Austragen, wurde zwischen der Darstellung von Figur 14 und Figur 15 der Stössel 69 ganz hineingedrückt, die Flüssigkeit 72 von Flüssigkeitsbehälter 62 in Pulverbehälter 61 transferiert, der Stössel 69 abgezogen dann zwischen Kolben 63 und Unterseite von Drehknopf 66 eingeklemmt derart, dass durch Drücken des Mischstabes dieser über den Stössel 69 auf den Kolben 63 wirkt, um das Gemisch 71+72 auszutragen. Dabei kann der Stössel mit einem etwas über-halbringförmigen Querschnitt ausgebildet sein, der beim Einklemmen dem Mischstab teilweise umfasst und diesen durch das Profil klemmt.

In der Darstellung gemäss den Figuren 14 und 15 wurde die Ventilanordnung 73 nur summarisch gezeichnet und beschrieben, um das Wirken des losen Stössels zu beschreiben. Die detaillierte Beschreibung der Ventilanordnung 73 erfolgt anhand der Figuren 16 - 19.

Die Austragvorrichtung 60 gemäss den Figuren 16 bis 19 kann vor allem auch dazu verwendet werden, eine Flüssigkeit wie beispielsweise Blut im Flüssigkeitsbehälter 62 aufzunehmen und nach Transfer in den Pulverbehälter mit dem Pulver zu mischen und anschliessend auszutragen. Dazu sind die Behälter über ein Dreiwegventil mit Drehring 100 miteinander oder mit dem gemeinsamen Ein/Auslass 101 verbunden.

Dabei ist der Stössel und das Kolbenende so gestaltet, dass der Stössel angekuppelt werden kann derart, dass mit dem Stössel der Kolben gezogen werden kann, um eine Flüssigkeit anzusaugen. Diese Kupplung kann beispielsweise durch das Vorsehen eines beabstandeten umlaufenden Abvsatz mit etrwas kleineren Durch messer als der Kolben beim stösselseitigen Kolbenende verwirklicht werden, um den eine Innennut am kolbenseitigen Ende des etwas über-halbringförmigen Stössels durch Drehen eingeführt oder ausgeklinkt werden kann.

Die als Dreiweg-Ventil ausgebildete Ventilanordnung 73 besteht im wesentlichen aus der Befestigungsteil 76, einem daran befestigten Ventilkörper 81 und einem Drehring 100. Der Ventilkörper 81 weist ein über die Anschlussstutzen 102 und 103 in die beiden Behälterauslässe 104 und 105 steckbares Anschlussteil 106 auf, das ausgangsseitig in das Ein/ Auslassteil 107 übergeht. Das Anschlussteil 106 ist mittels einem umlaufenden Kragen 114 in einer umlaufenden Nut 115 des Befestigungsteils 76 gehalten.

Das Ein/Auslassteil 107 weist eine Bohrung 109 auf, die abgewinkelt ist und an der Peripherie des Ein/Auslassteils mündet. Analog dazu münden die Ein/Auslässe 110, 111 des Anschlussteils 106 des Ventilkörpers ebenfalls an der Peripherie, wie dies am besten aus Figur 17 hervorgeht und zwar auf gleicher Höhe wie die Mündung der Bohrung 109 im Ein/Auslass. Um das Ein/Auslassteil 107 ist der am Anschlussteil 106 gehaltene Drehring 100 angeordnet, wobei der Drehring bis über die peripheren Ein/Ausgänge der Ein/Auslässe reicht und eine sich etwas über einen Drittel des Umfanges erstreckende Innennut 112 aufweist, wie dies aus den Schnitten A, B, C, D hervorgeht.

Wie ein Vergleich der Figuren 16 bis 19, bzw. A, B, C und D zeigt, verbindet die Innennut 112 je nach Stellung des Drehrings entweder den Flüssigkeitsbehälter 62 mit dem gemeinsamen Ein/Auslass 101, Figur 16, A oder die beiden Ein/Auslässe 110 und 111 miteinander, Figur 17, B oder den Pulverbehälter 61 mit dem gemeinsamen Ein/Auslass 100, Fig. 18, C oder der Drehring ist in einer geschlossenen Stellung, D, Fig. 19, ohne Verbindung zu einem Ein- oder Auslass.

In der Stellung von Figur 16 kann eine Flüssigkeit, z.B. Blut angesaugt werden, indem der Kolben 68 hochgezogen wird. In der Stellung von Figur 17 wurde der Drehring um 120° gedreht, so dass die beiden Behälter miteinander verbunden sind und die Flüssigkeit in den Pulverbehälter transferiert werden kann. Nach weiterer Drehung des Drehrings um 60° können beide Behälter verschlossen werden und das Gemisch vermischt werden. Anschliessend wird die Ventilanordnung in die Stellung von Figur 18 gebracht, so dass das Gemisch von Pulverbehälter 61 ausgetragen werden kann. Es ist auch möglich, eine weitere Flüssigkeit in den Flüssigkeitsbehälter anzusaugen, in den Pulverbehälter zu transferieren und anschliessend zu vermischen.

Der Ein/Auslass 101 ist als Luer-Anschluss mit Gewinde 108 ausgebildet, an dem entweder eine weitere Spritze oder ein Auslassteil angeschlossen werden kann. Es ist jedoch auch möglich, die Ventilanordnung mittels dem Bajonettanschluss zu lösen und statt dessen ein anderes Anschlussteil, zB. einen weiteren, an sich üblichen Luer-Anschluss 31, siehe Fig. 20, mittels der Bajonettverbindung zu befestigen.

Die Ventilanordnung 73 kann auch für andere Vorrichtungen als die Beschriebenen verwendet werden, d.h. ganz allgemein als Dreiweg-Ventil mit Drehring mit teilweise umlaufender Innennut, um zwei Behälter-Ein/Auslässe entweder miteinander oder je mit einem gemeinsamen Ein/Auslass zu verbinden oder alle Ein/Auslässe zu verschliessen. Auch bei einer solchen Ausführung kann die Befestigung der Ventilanordnung auch auf andere Weise als mit Bajonettsanschlussteilen erfolgen, z.B. mittels einer drehfesten Schnappverbindung, die auch direkt, ohne Befestigungsteil, am Ventilkörper angeordnet sein kann.

Es sind noch weitere Ventilanordnungen möglich, so z. B. eine selbsttätige Ventilanordnung oder ein einfaches Drehventil oder flachabdichtendes Drehventil mit einem Drehknopf mit Nut, die entweder eine Verbindung herstellen oder verschliessen.

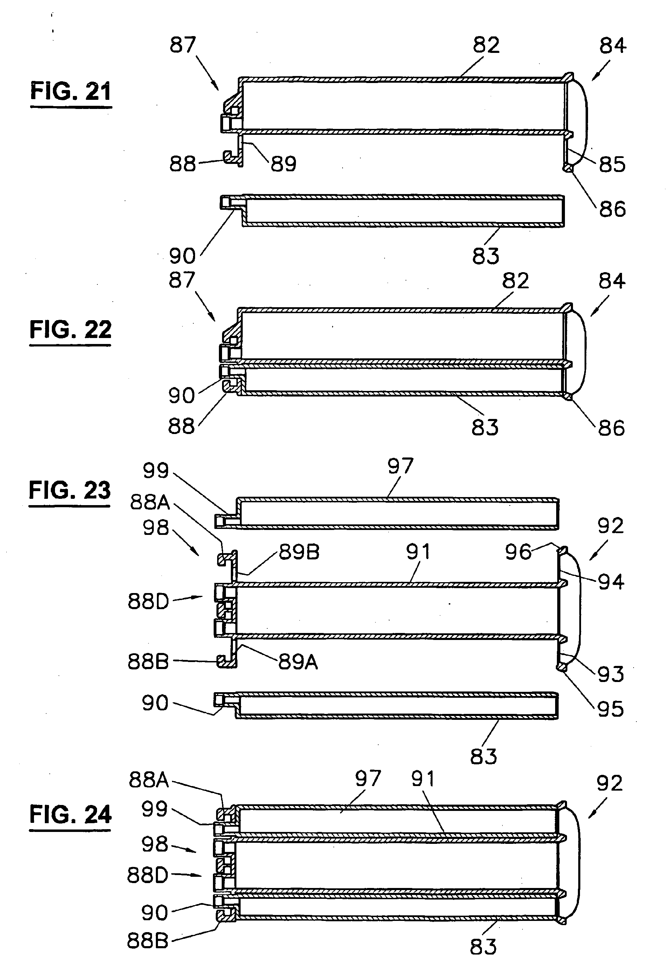

Aus den Figuren 21 - 24 geht hervor, dass die Mehrkomponentenspritzen oder -kartuschen nicht jeweils einstückig sondern als vereinzelte Vorratsbehälter gefertigt und gefüllt werden können, um bei Gebrauch und Bedarf zusammengefügt zu werden.

In den Figuren 21 und 22 sind ein Pulverbehälter 82 und ein Flüssigkeitsbehälter 83 dargestellt, wobei der Behälter mit grösserem Querschnitt, Pulverbehälter 82, einen Rückhalteflansch 84 aufweist, dessen den Behälter 82 überragender Teil 85 dazu dient, den zweiten Behälter 83 aufzunehmen. Dazu weist der überragende Teil 85 über seinen Umfang einen umlaufenden Wulst 86 auf.

Auslassseitig weist der grössere Behälter einen Auslassflansch 87 auf, der die üblichen Bajonett-Anschlussmittel 88 aufweist sowie eine Öffnung 89, um den Auslass 90 des kleineren Behälters aufzunehmen. Die beschriebene Anordnung gilt auch für 1:1-Behälter.

Sinngemäss gilt dies auch für eine Dreikomponentenspritzen-Anordnung gemäss den Figuren 23 und 24. Der Pulverbehälter 91 weist einen Rückhalteflansch 92 mit zwei überragenden Teilen 93 und 94 auf, die je einen um Teil 93, bzw. 94 umlaufenden Wulst 95 und 96 aufweisen, die dem Festhalten des Flüssigkeitsbehälters 83, bzw. 97 dienen. Der Auslassflansch 98 weist zwei Bajonett-Anschlussklauen 88A und 88B und eine zentrale Bajonettdoppelklaue 88D sowie zwei Öffnungen 89A und 89B auf, um die Auslässe 90, bzw. 99 der Flüssigkeitsbehälter aufzunehmen. An den beiden Bajonett-Anschlüssen können entweder die Behälter 91 und 83 oder 91 und 97 angeschlossen werden, die je entweder mit einer Ventilanordnung oder einem Ausgangsteil oder einem Mischer versehen sein können.

In den gezeigten Ausführungsbeispielen wird der Auslass des Flüssigkeitbehälters in die Öffnung 89A oder 89B gesteckt und der Behälter über den Wulst 86 oder 95, 96 hinweg eingeschnappt. Die Verbindung und Befestigung der einzelnen Behälter zu einer Doppel- oder Drei- oder Mehrkomponenten-Austragvorrichtung kann auch über andere Mittel erfolgen, z.B mittels Schnappverbindungen oder dergl. Auch kann eine Doppelspritze oder Doppelkartusche auf diese Weise mit einem weiteren, vereinzelten Behälter verbunden werden.

## Patentansprüche

1. Vorrichtung zum Aufbewahren, Mischen und Austragen von Komponenten, mit Mitteln zum Vermischen einer ersten Komponente (27, ) mit einer zweiten, flüssigen Komponente (28, ) und zum Austragen des vermischten Materials, wobei die einzelnen Komponenten (27, 28, 52, ) in je einem nebeneinander liegenden Behälter (2, 3, 44; ) angeordnet sind und im Transferbereich (7, ) zwischen dem Auslassbereich (26) des Behälters (3, ) zum Aufbewahren der zweiten, flüssigen Komponente (28, ) und dem Flüssigkeitseinlass (25, 78) des Behälters (2, ) zum Aufbewahren der ersten Komponente (27, ) wahlweise ein Verschluss oder ein Verbindungskanal vorhanden ist, wobei die Mittel zum Vermischen eine im Behälter (2, ) für die erste Komponente (27) angeordnete Mischeinrichtung (6, ) enthalten, die von den Austragmitteln (4, ) für das Gemisch (27 + 28; ) getrennt ist und einen im Behälter hin- und herbewegbaren und drehbaren Mischstab (9, ) mit einem Mischorgan (11, ) aufweist, **dadurch gekennzeichnet, dass** der Mischstab (9) eine Sollbruchstelle (12, 59) aufweist.

2. Vorrichtung nach Anspruch 1, **dadurch gekennzeichnet, dass** das Mischorgan eine gelochte und/oder mit Ausnehmungen am Umfang versehene Mischscheibe (11, ) ist.

3. Vorrichtung nach Anspruch 1, **dadurch gekennzeichnet, dass** im Transferbereich (7, ) eine Ventilanordnung (8, 73) angeordnet ist, um wahlweise den Verschluss oder die Verbindung herzustellen.

4. Vorrichtung nach Anspruch 3, **dadurch gekennzeichnet, dass** die Ventilanordnung (8) eine Ventilkappe (17) ist, die abnehmbar am Gehäuseboden (53, 54) der Behälter (2, 3) für die erste und zweite, flüssige Komponente befestigbar ist und zwei Paare Zapfen (21, 22) aufweist, wovon ein Paar volle Zapfen (21) und das andere Paar (22) über den Verbindungkanal (24) miteinander verbundene Zapfen sind, wobei die Ventilkappe derart befestigbar ist, dass die Zapfen die Verbindung im Transferbereich (7) entweder offen lässt oder unterbricht.

5. Vorrichtung nach Anspruch 4, **dadurch gekennzeichnet, dass** die Ventilkappe (17) eine gegenüber dem Kappengehäuse (18) drehbar angeordnete Zapfenanordnung (19) mit den vollen (21) und miteinander verbundenen (22) Zapfenpaaren aufweist.

6. Vorrichtung nach Anspruch 3, **dadurch gekennzeichnet, dass** die Ventilanordnung (73) ein Dreiwegventil mit einen Drehring (100) mit einer über einen Teil des Umfanges umlaufenden Innennut (112) aufweist, mit dem wahlweise eine Bohrung (109) im gemeinsamen Ein/ Auslassteil (107) mit einem der Ein/Auslässe (104, 105) der Behälter oder beide Ein/Auslässe (104, 105) der Behälter miteinander verbindbar sind, oder alle Ein/Auslässe verschlossen sind.

7. Vorrichtung nach Anspruch 6, **dadurch gekennzeichnet, dass** das Dreiwegventil einen Ventilkörper (81) aufweist, an dem behälterseitig ein Befestigungsteil (76) angeordnet ist und an dem auslassseitig der Drehring (100) befestigt ist.

8. Vorrichtung nach einem der Ansprüche 1 bis 7, **dadurch gekennzeichnet, dass** die erste Komponente ein pulver-, granulatförmiges oder poröses Material (27) ist.

9. Vorrichtung nach einem der Ansprüche 1 bis 8 zum Mischen unter Vakuum, **dadurch gekennzeichnet, dass** der Behälter (3) für die zweite, flüssige Komponente am Einlassende (36) einen Bereich mit grösserem Durchmesser (D2) als der Durchmesser (D1) des übrigen Behälters aufweist.

10. Vorrichtung nach einem der Ansprüche 1 bis 9, **dadurch gekennzeichnet, dass** der Kolben (14, 68)) für die zweite, flüssige Komponente (28) entweder mit einem Stössel (15, ) beaufschlagt oder durch Unterdruck bewegbar ist.

11. Vorrichtung nach einem der Ansprüche 1 bis 10, **dadurch gekennzeichnet, dass** das gemeinsame Ein/Auslassteil (107) der Ventilanordnung (73) mit einem Anschluss-Zubehörteil (94, 95) oder -element (108) versehen ist, um eine Spritze oder ein anderes Teil anschliessen zu können.

12. Vorrichtung nach einem der Ansprüche 1 bis 11, **dadurch gekennzeichnet, dass** die Vorrichtung (42) mindestens einen weiteren Behälter (44) für eine flüssige Komponente (52) enthält und der Auslass (48) für das Gemisch, gebildet aus der ersten Komponente und der zweiten, flüssigen Komponente, von Behälter (2) und der Auslass (49) für die weitere flüssige Komponente (52), mit einem gemeinsamen Verschluss (55) versehen sind und die beiden Auslässe (48, 49) einen gemeinsamen Anschluss (47) für einen Mischer (30) oder ein Zubehör bilden.

13. Vorrichtung nach einem der Ansprüche 1 bis 12, **dadurch gekennzeichnet, dass** die Behälter (82, 83; 91, 83, 97) als vereinzelte, zusammenfügbare Teile ausgeführt sind.

14. Vorrichtung nach Anspruch 13, **dadurch gekennzeichnet, dass** der erste Behälter (82, 91) einen Rückhalteflansch (84, 92) aufweist, der einen oder zwei den Behälter überragende(n) Teil(e) (85;, 93, 94) mit daran umlaufenden Wulst (86; 95, 96) aufweist, um den zweiten (83) oder den zweiten und dritten Behälter (83, 97) aufzunehmen, wobei die Auslässe (90, 98) der anderen Behälter (83, 97) durch Öffnungen (89, 89A, 89B) im Auslassflansch (87, 98) des ersten Behälters (82, 91) steckbar sind.

15. Vorrichtung nach einem der Ansprüche 1 bis 14, **dadurch gekennzeichnet, dass** der Auslassflansch der Behälter (2, 3; 82, 83; 91, 97, 83) codierte Bajonett-Anschlussmittel (16, 20; 18A; 77, 88; 88A, 88B 88D) aufweist.

16. Vorrichtung nach einem der Ansprüche 1 bis 15, **dadurch gekennzeichnet, dass** die Mischeinrichtung durch die Austragmittel (4, 63) für das Gemisch (27 + 28) geführt ist.

17. Vorrichtung nach Anspruch 7, **dadurch gekennzeichnet, dass** das Befestigungsteil (76) des Dreiweg-Ventils codierte Bajonett-Anschlussmittel (77) oder Schnappverbindungsmittel aufweist.

18. Vorrichtung nach Anspruch 6, **dadurch gekennzeichnet, dass** das Dreiwegventil einen Ventilkörper aufweist, an dem behälterseitig eine Schnappverbindung und auslassseitig der Drehring angeordnet ist.

19. Verfahren zum Aufbereiten und Austragen eines Gemisches aus einer ersten Komponente und mindestens einer zweiten, flüssigen Komponente, mit einer Anordnung gemäss Anspruch 14, **dadurch gekennzeichnet, dass** die zweite, flüssige Komponente über den Verbindungskanal in die erste Komponente eingebracht und anschliessend mit ihr vermischt wird und das Gemisch sowie eine dritte, flüssige Komponente durch einen am Auslass des Behälters der ersten Komponente und am Auslass der dritten, flüssigen Komponente angeschlossenen Mischer oder Zubehör ausgetragen wird.

## Claims

1. A device for storing, mixing, and dispensing components, comprising means for mixing a first component (27) with a second, liquid component (28) and for dispensing the mixed material, wherein the individual components (27, 28, 52) are disposed in one container (2, 3, 44) each, the containers being arranged side by side, and either a closure or a connecting channel is provided in the transfer area (7) between the outlet area (26) of the container (3) for storing the second, liquid component (28) and the liquid inlet (25) of the container (2) for storing the first component (27), wherein the means for mixing comprise a mixing arrangement (6) arranged in the container (2) for the first component (27), the mixing arrangement being separated from the dispensing means (4) for the mixture (27 + 28) and comprising a mixing rod (9) with a mixing member (11), the mixing rod being movable back and forth and rotatable in the container, **characterized in that** the mixing rod (9) has a predetermined breaking point (12, 59).

2. The device according to claim 1, **characterized in that** the mixing member is a mixing disk (11) that is perforated and/or provided with peripheral cutouts.

3. The device according to claim 1, **characterized in that** in the transfer area (7) a valve assembly (8, 73) is arranged in order to selectively provide the closure or connection.

4. The device according to claim 3, **characterized in that** the valve assembly (8) is a valve cap (17) that is removably attachable to the enclosure bottom (53, 54) of the containers (2, 3) for the first and the second liquid components and comprises two pairs of plugs (21, 22) of which one pair are solid plugs (21) and the other pair (22) are plugs that are connected to each other by the connecting channel (24), the valve cap being attachable such that the plugs either leave open or interrupt the connection in the transfer area (7).

5. The device according to claim 4, **characterized in that** the valve cap (17) comprises a plug arrangement (19) of the solid (21) and the interconnected (22) plug pairs that is arranged rotatably with respect to the cap enclosure (18).

6. The device according to claim 3, **characterized in that** the valve assembly (73) comprises a three-way valve having a rotary ring (100) with a circular internal groove (112) extending over a part of the circumference that allows to selectively connect one bore (109) in the common inlet/outlet portion (107) to one of the inlets/outlets (104, 105) of the containers, or both inlets/outlets (104, 105) of the containers to each other, or to close all inlets/outlets.

7. The device according to claim 6, **characterized in that** the three-way valve comprises a valve body (81) having a fastening portion (76) arranged on its container side and the rotary ring (100) secured to its outlet side.

8. The device according to any one of claims 1 to 7, **characterized in that** the first component is a powdery, granular, or porous material (27).

9. The device according to any one of claims 1 to 8 for mixing under vacuum, **characterized in that** at its inlet end (36), the container (3) for the second, liquid component comprises a section having a greater diameter (D2) than the diameter (D1) of the rest of the container.

10. The device according to any one of claims 1 to 9, **characterized in that** the piston (14, 68) for the second, liquid component (28) is either actuated by a thrust rod (15) or movable by negative pressure.

11. The device according to any one of claims 1 to 10, **characterized in that** the common inlet/outlet portion (107) of the valve assembly (73) is provided with a coupling accessory (94, 95) or element (108) that allows the connection of a syringe or another part.

12. The device according to any one of claims 1 to 11, **characterized in that** the device (42) comprises at least another container (44) for a liquid component (52), and **in that** the outlet (48) for the mixture composed of the first component and the second, liquid component from container (2) and the outlet (49) for the additional liquid component (52) are provided with a common closure (55), and **in that** the two outlets (48, 49) form a common coupling (47) for a mixer (30) or an accessory.

13. The device according to any one of claims 1 to 12, **characterized in that** the containers (82, 83; 91, 83, 97) are in the form of singulated parts that can be assembled.

14. The device according to claim 13, **characterized in that** the first container (82, 91) comprises a retaining flange (84, 92) with one or two part(s) (85; 93, 94) extending beyond the container and provided with circular bead(s) (86; 95, 96) for receiving the second (83) or the second and third containers (83, 97), the outlets (90, 98) of the other containers (83, 97) being adapted to be pushed through openings (89, 89A, 89B) in the outlet flange (87, 98) of the first container (82, 91).

15. The device according to any one of claims 1 to 14, **characterized in that** the outlet flange of the containers (2, 3; 82, 83; 91, 97, 83) is provided with coded bayonet coupling means (16, 20; 18A; 77, 88; 88A, 88B, 88D).

16. The device according to any one of claims 1 to 15, **characterized in that** the mixing arrangement is passed through the dispensing means (4, 63) for the mixture (27 + 28).

17. The device according to claim 7, **characterized in that** the fastening portion (76) of the three-way valve comprises coded bayonet coupling means (77) or snap-on connecting means.

18. The device according to claim 6, **characterized in that** the three-way valve comprises a valve body having a snap-on connection arranged on its container side and the rotary ring arranged on its outlet side.

19. A method for conditioning and dispensing a mixture of a first component and at least a second, liquid component by means of the device of claim 14, **characterized in that** the second, liquid component is introduced into the first component via the connecting channel and subsequently mixed therewith, and **in that** the mixture is dispensed along with a third, liquid component through a mixer or accessory that is connected to the outlet of the container of the first component and to the outlet of the third, liquid component.

## Revendications

1. Dispositif de conservation, de mélange et d'extraction de composants, doté de moyens permettant de mélanger un premier composant (27) avec un deuxième composant (28) liquide et d'extraire le matériau mélangé,
les différents composants (27, 28, 52) étant disposés dans des récipients respectifs (2, 3, 44) situés les uns à côté des autres,
une fermeture ou un canal de liaison étant prévus sélectivement dans la zone de transfert (7) entre la zone de sortie (26) du récipient (3) de conservation du deuxième composant liquide (28) et l'entrée de liquide (25, 78) du récipient (2) de conservation du premier composant (27),
les moyens de mélange contenant un dispositif de mélange (6) disposé dans le récipient (2) prévu pour le premier composant (27) et séparé des moyens d'extraction (4) prévus pour le mélange (27 + 28) et présentant un barreau de mélange (9) doté d'un organe de mélange (11) apte à tourner et à se déplacer en va- et-vient dans le récipient,
**caractérisé en ce que**
le barreau de mélange (9) présente un emplacement (12, 59) de rupture préférentielle.

2. Dispositif selon la revendication 1, **caractérisé en ce que** l'organe de mélange est une plaque de mélange (11) perforée et/ou dotée de découpes à sa périphérie.

3. Dispositif selon la revendication 1, **caractérisé en ce qu'**un ensemble de soupape (8, 73) est disposé dans la zone de transfert (7) pour établir sélectivement la fermeture ou la liaison.

4. Dispositif selon la revendication 3, **caractérisé en ce que** l'ensemble de soupape (8) est un bonnet de soupape (17) fixé de manière amovible sur le fond (53, 54) du boîtier des récipients (2, 3) prévus pour le premier composant et le deuxième composant liquide et présente deux paires de tourillons (21, 22), dont une paire est constituée de tourillons pleins (21) et l'autre paire (22) de tourillons reliés l'un à l'autre par le canal de liaison (24), le bonnet de soupape pouvant être fixé de telle sorte que le tourillon laisse ouverte ou interrompt la liaison dans la zone de transfert (7).

5. Dispositif selon la revendication 4, **caractérisé en ce que** le bonnet de soupape (17) présente un ensemble de tourillon (19) disposé à rotation par rapport au boîtier de bonnet (18) et présentant les paires de tourillons pleins (21) et de tourillons (22) reliés l'un à l'autre.

6. Dispositif selon la revendication 3, **caractérisé en ce que** l'ensemble de soupape (73) présente une soupape à trois voies dotée d'une bague rotative (100) présentant une rainure intérieure (112) qui s'étend sur une partie de sa périphérie et par laquelle un alésage (109) ménagé dans la partie commune d'entrée ou de sortie (107) permet de relier l'une à l'autre l'une des entrées ou sorties (104, 105) des récipients ou les deux entrées/sorties (104,105) des récipients, ou de fermer toutes les entrées/sorties.

7. Dispositif selon la revendication 6, **caractérisé en ce que** la soupape à trois voies présente un corps de soupape (81) sur lequel une pièce de fixation (76) est disposée côté récipient et sur lequel la bague rotative (100) est fixée côté sortie.

8. Dispositif selon l'une des revendications 1 à 7, **caractérisé en ce que** le premier composant est un matériau pulvérulent, granulé ou poreux (27).

9. Dispositif selon l'une des revendications 1 à 8, destiné à assurer un mélange sous vide, **caractérisé en ce que** le récipient (3) prévu pour le deuxième composant liquide présente à l'extrémité d'entrée (36) une partie dont le diamètre (D2) est plus grand que le diamètre (D1) du reste du récipient.

10. Dispositif selon l'une des revendications 1 à 9, **caractérisé en ce que** le piston (14, 68) prévu pour le deuxième composant liquide (28) peut être déplacé soit en étant sollicité par un poussoir (15) soit sous dépression.

11. Dispositif selon l'une des revendications 1 à 10, **caractérisé en ce que** la partie commune d'entrée/sortie (107) de l'ensemble de soupape (73) est dotée d'une pièce auxiliaire (94, 95) ou d'un élément (108) de raccordement qui permet de raccorder une aiguille ou une autre pièce.

12. Dispositif selon l'une des revendications 1 à 11, **caractérisé en ce que** le dispositif (42) contient au moins un autre récipient (44) pour un composant liquide (52), **en ce que** la sortie (48) prévue pour le mélange formé du premier composant et du deuxième composant liquide provenant du récipient (2) et la sortie (49) pour l'autre composant liquide (52) sont dotés d'une fermeture commune (55) et **en ce que** les deux sorties (48, 49) forment un raccordement commun (47) pour un mélangeur (30) ou un accessoire.

13. Dispositif selon l'une des revendications 1 à 12, **caractérisé en ce que** les récipients (82, 83; 91, 83, 97) sont configurés comme pièces distinctes aptes à être assemblées.

14. Dispositif selon la revendication 13, **caractérisé en ce que** le premier récipient (82, 91) présente une bride de retenue (84, 92) qui présente une ou deux parties (85; 93, 94) qui débordent du récipient et sur lesquelles est prévu un bourrelet périphérique (86; 95, 96), pour reprendre le deuxième récipient (83) ou le deuxième et le troisième récipient (83, 97), les sorties (90, 98) des autres récipients (83, 97) pouvant être enfichées par des ouvertures (89, 89A, 89B) dans la bride de sortie (87, 98) du premier récipient (82, 91).

15. Dispositif selon l'une des revendications 1 à 14, **caractérisé en ce que** la bride de sortie des récipients (2, 3; 82, 83; 91, 97, 83) présente des moyens codés de raccordement à baïonnette (16, 20; 18A; 77, 88; 88A, 88B, 88D).

16. Dispositif selon l'une des revendications 1 à 15, **caractérisé en ce que** le dispositif de mélange est guidé par les moyens d'extraction (4, 63) prévus pour le mélange (27 + 28).

17. Dispositif selon la revendication 7, **caractérisé en ce que** la pièce de fixation (76) de la soupape à trois voies présente des moyens codés de raccordement à baïonnette (77) ou des moyens de raccordement rapide.

18. Dispositif selon la revendication 6, **caractérisé en ce que** la soupape à trois voies présente un corps de soupape sur lequel une liaison rapide est disposée côté récipient et une bague rotative est disposée côté sortie.

19. Procédé de préparation et d'extraction d'un mélange constitué d'un premier composant et d'au moins un deuxième composant liquide, à l'aide d'un agencement selon la revendication 14, **caractérisé en ce que** le deuxième composant liquide est apporté par le canal de liaison dans le premier composant et est ensuite mélangé avec lui, et **en ce que** le mélange ainsi qu'un troisième composant liquide sont extraits par un mélangeur ou un accessoire raccordé à la sortie du récipient prévu pour le premier composant et à la sortie du troisième composant liquide.
